# EUROPÄISCHE PATENTANMELDUNG

(11) **EP 2 952 578 A1**
(43) Veröffentlichungstag der Anmeldung: **09.12.2015**
(21) Anmeldenummer: 14171027.7
(22) Anmeldetag: 03.06.2014
(51) Int. Cl.: C12N 5/073, C12N 5/0775, C12N 5/077, C12N 5/0793

(54) **Epigenetische Reprogrammierung von fötalen Stammzellen**

(71) Anmelder: Wernet, Peter, 40627 Düsseldorf (DE); Santourlidis, Simeon, 40225 Düsseldorf (DE); Ghanjati, Foued, 40225 Düsseldorf (DE)
(72) Erfinder: WERNET, Peter, 40627 Düsseldorf (DE); SANTOURLIDIS, Simeon, 40225 Düsseldorf (DE); GHANJATI, Foued, 40225 Düsseldorf (DE)
(74) Vertreter: Von Kreisler Selting Werner - Partnerschaft von Patentanwälten und Rechtsanwälten mbB

(57) **Zusammenfassung**

Verfahren zur Erzeugung einer spheroiden multipotenten somatischen Stamm-zelle umfassend die Schritte:
- Bereitstellen einer fötalen somatischen Stammzelle
- Behandlung der fötalen somatischen Stammzelle mit mindestens einem epigenetisch wirksamen Reagenz, wodurch eine spheroide multipotente somatische Stammzelle erzeugt wird.

## Beschreibung

Die vorliegende Erfindung betrifft multipotente somatische Stammzellen.

Dem Fachmann sind verschiedene Formen von Stammzellen bekannt.

Embryonale Stammzellen sind Zellen, die *in vivo* und *in vitro* in der Lage sind, sich in alle Körperzellen auszudifferenzieren. Die Ausdifferenzierung embryonaler Stammzellen verläuft über bereits weiter ausdifferenzierte Stammzellen, die keine pluripotenten, sondern nur multipotente Eigenschaften haben.

Eine Gruppe dieser teilweise ausdifferenzierten Zellen sind mesenchymale Stammzellen (MSC); siehe beispielsweise Erices et al., British Journal of Hematology 109 (2000), Seiten 235-242. Sie sind die Vorläuferzellen des Bindegewebes.

Eine weitere Stammzelle, die multipotent ist, ist die unrestringierte somatische Stammzelle (USSC), wie sie beispielsweise in der WO 2002/036751 beschrieben ist.

Während MSC unter geeigneten Bedingungen in Adipocyten, Chondrocyten, oder Osteoblasten ausdifferenziert werden können, ist eine Ausdifferenzierung in Cardiomyocyten und neuronale Zellen nicht möglich. USSC können hingegen nicht in Adipocyten, aber in Hepatocyten und Cardiomyocyten sowie in Chondrozyten und Osteoblasten ausdifferenziert werden.

Aufgabe der vorliegenden Erfindung war es, weitere Stammzellen bereitzustellen, die interessante weitere Eigenschaften aufweisen.

Gelöst wird die Aufgabe durch ein Verfahren mit den Merkmalen des Anspruchs 1.

Erfindungsgemäß umfasst das Verfahren folgende Schritte:
- Bereitstellen einer fötalen somatischen Stammzelle
- Behandlung der fötalen somatischen Stammzelle mit mindestens einem epigenetisch wirksamen Reagenz, wodurch eine spheroide multipotente somatische Stammzelle erzeugt wird

Das Bereitstellen von fötalen somatischen Stammzelle (FSC) ist dem Fachmann aus dem Stand der Technik bekannt, nicht jedoch, dass unter dem Einfluss von epigenetisch wirksamen Reagenzien eine Änderung des Methyloms, eine Änderung der Genexpression und auch des Phenotyps erfolgt.

Fötale somatische Stammzellen (FSC) können in besonders einfacher Weise aus Nabelschnurblut gewonnen werden.

Auffällig ist insbesondere, dass die Zellen unter der Behandlung spheroid werden und in andere Zellen als die FSC differenziert werden können. Die erhaltenden Zellen werden auch als reprogrammierte fötale StammZellen bezeichnet (rFSC). Bevorzugt werden humane Zellen eingesetzt, die dann als HFSC bzw rHFSC bezeichnet werden.

Als epigenetisch wirksames Reagenz sind Reagenzien geeignet, die beispielsweise DNA demethylierende Eigenschaften haben oder Histon-acetylierende Eigenschaften haben. Beispiele hiervon sind beispielsweise 5'-Aza-2-deoxycytidin, Trichostatin-A, 5-Azacytidin, Valproinsäure, BIX-01294, Dznep und Mischungen davon.

Gegenstand der Erfindung ist auch eine spheroide multipotente somatische Stammzelle, die durch das erfindungsgemäße Verfahren erhältlich ist. Im Gegensatz zu einer USSC-Vorläuferzelle, der USSC, können diese beispielsweise in Adipocyten differenziert werden. Im Gegensatz zu einer Vorläuferzelle MSC können diese in Cardiomyocyten bzw neuronale Zellen differenziert werden. Allgemein ist eine Differenzierung der rFSC zu Adipocyten, Cardiomyocyten, Chondrocyten, Osteoblasten und neuronalen Zellen möglich.
Figur 1:
   - Foto 1 zeigt FSC, die als adhärente Monolayer wachsen und eine spindelförmige Morphologie mit einer Größe der Einzelzelle von 20 bis 25 µm aufweisen.
   - Foto 2 zeigt die gemäß dem Beispiel entstehenden pre-spheroiden FSC Kolonien, die durch epigenetische Behandlung der FSC erhalten werden.
   - Foto 3 zeigt die daraus entstehenden spheroiden Zellen.
   - Foto 4a zeigt Adipozyten (Fettzellen), die spontan aus den spheroiden Zellen entstehen.
   - Foto 4b zeigt die Differenzierung.
   - Foto 4c zeigt die Fettzellen unter spezifischer Anfärbung.
Figur 2A zeigt die erfindungsgemäßen spheroiden Stammzellen. Diese wurden mit Hilfe des Vektors aus Figur 2B in GFP (Green Fluorescent Protein) exprimierende spheroiden rFSC überführt (Figur 2C)
Figur 2D zeigt die Differenzierung zu Cardiozyten.
Figur 3 zeigt eine epigenetische Untersuchung.
Figur 5 zeigt die Proliferation der spheroiden rFSC Zellen in Kultur.
Figur 6 zeigt die Seneszenz-assoziierte Expression von ß-Galaktosidase nach Passage 8.
Figur 7 zeigt die Seneszenz-assoziierte Expression von ß-Galaktosidase nach Passage 30.
Figur 8 zeigt den Nachweis von langen Teleomeren mittels TeloTAGGG Telomere Length Assay.
Figur 9 zeigt den Nachweis von langen Teleomeren mittels PCR.
Figur 10 zeigt einen PCR Nachweis eines Telomerase Transkripts.

Die Erfindung wird durch die nachfolgenden Beispiele näher erläutert.

### Beispiel 1: Gewinnung von FSC Zelllinien

Die Gewinnung und Kultivierung der FSC Zelllinien erfolgte wie in Koegler et al. (J Exp Med. 2004 Jul 19;200(2):123-35. A new human somatic stem cell from placental cord blood with intrinsic pluripotent differentiation potential und Exp Hematol. 2006 Nov;34(11):1589-95. Comparative generation and characterization of pluripotent unrestricted somatic stem cells with mesenchymal stem cells from human cord blood*)* und Santourlidis (Stem Cell Res. 2011 Jan;6(1):60-9. Unrestricted somatic stem cells (USSC) from human umbilical cord blood display uncommitted epigenetic signatures of the major stem cell pluripotency genes*)* beschrieben.

Die Kultivierung erfolgte in
- DMEM (low glucose)
- 30% FCS (Perbio)
- 1% Pen/Strep. L-Glutamin.

Die Kulturbedingungen sind in allen Beispielen 37°C und 5% CO₂.

### Beispiel 2: Erzeugung der spheoroiden multipotenten, unrestringierten somatischen Stammzellen (rFSC)

5-Aza-2'-deoxycytidine (Aza) (auch Decitabine genannt) ist ein epigenetisches wirksames Reagenz, das die DNA-Methyltransferase-Aktivität hemmt, was zu DNA-Demethylierung und in vielen Fällen zur Genaktivierung führt.

Trichostatin A (TSA) ist ein Streptomyces Metabolit, der spezifisch die Histon-Deacetylase von Säugetieren hemmt. Dies führt zu einer Acetylierung von Histonen und in einigen Fällen zu Genaktivierung.

### Erzeugungsmedium für prespheroide FSC:

- DMEM 20% FCS
- 2 mM L-Glutamat
- 0,1 mM µM ß-Mercaptoethanol
- 1% Penicillin/Streptomycin
- 100 ng/ml Recombinant Human Stem Cell Factor (Fa. Immuno tools).

### Epigenetischen Behandlung der FSC mit Aza und TSA-Erzeugung von prespheroiden USSC

Auf 6-Well Zellkulturplatten werden am Tag 1 100.000 FSC Zellen nach der sechsten Passage auf die sechs Wells verteilt. Am zweiten Tag erreichen diese 60 bis 70% Konfluenz. Am dritten Tag wird das Medium gewechselt und dem oben erwähnten Erzeugungsmedium zusätzlich 1,5 µM Aza und 25 nM TSA zugesetzt. Das Medium wird an den Tagen 4 und 5 gewechselt, wobei jeweils das für Tag 3 beschriebene Medium eingesetzt wird. 50.000 bis 100.000 epigenetisch behandelte FSC wurden in neue 6-Well Kulturplatten überführt und anschließen in dem oben verwendeten Erzeugungsmedium (ohne Zusatz von Aza und TSA) kultiviert, wobei alle drei Tage das Medium gewechselt wird. Prespheroide FSC Kolonien erscheinen nach etwa 10 bis 14 Tage.

### Spheroid-Medium:

- 390mL: Knockout DMEM/F12 (Fa. Gibco)
- 100mL: Knockout serum replacement (Fa. Gibco)
- 5mL: Non-essential amino acids
- 5mL: Pen/Strep/Glu
- 100µM: β-Mercaptoethanol

### Bildung der spheroiden Zellen:

Die gebildeten prespheroiden Kolonien können einzeln mit 200 µl Pipettenspitzen gepickt werden und werden dann in 6 cm Platten mit 3 ml des oben beschriebenen Spheroid-Mediums versetzt.

Jeweils nach 48 h wird das Medium durch ein 0,45 µm Filter filtriert und die Zellen in neuem Medium inkubiert, das zu 15 Vol.-% aus dem konditionierten und filtrierten vorherigen Medium besteht und zu 85% aus frischem Spheroid-Medium. Nach 1 bis 2 Wochen erscheint eine große Anzahl von spheroiden Zellen (rFSC).

### Beispiel 3: Induktion von spheroiden rFSCzu verschiedenen Zelltypen (multipotente Stammzelle)

### Beispiel 3.1: Differenzierung zu Adipozyten

Die spheroiden Zellen werden bis zu 70% Konfluenz herangezogen und in dem oben genannten Spheroid-Medium in Flaschen oder auf Platten kultiviert. Die Hälfte des Mediums wird alle drei Tage gewechselt. Adipozyten erscheinen nach 10 Tagen.

### Beispiel 3.2: Differenzierung zu Chondrozyten

Spheroide rFSC Zellen werden mit PBS Puffer gewaschen und bei 1500 rpm für 3 min zentrifugiert. Nach dem Waschen mit PBSW werden die Zellen mit einer Trypsin-Lösung (10fach) (mit NaCl/Na-EDTA) bei 37°C für 15 min inkubiert.

Die erhaltenen Pellets werden resuspendiert in Complete Culture Medium bestehend aus 90% Chondrogenic Differentiation Medium (erhältlich unter der Bestellnummer SH30889.02 von AdvanceSTEM) mit 10% AdvanceSTEM Cell Growth Medium (SH30878.02). Die Zellen werden bis zu 70% Konfluenz in Petrischalen herangezogen und weiter kultiviert. 50% des Mediums werden jeden dritten Tag während ein- bis zweiwöchigen Differenzierungsperiode gewechselt. Chondrozytenartige Zellen, die SOX9 exprimieren, können nach etwa sieben Tagen detektiert werden.

### Beispiel 3.3: Differenzierung zu Osteoblasten

Spheroide rFSC Zellen werden mit PBS gewaschen und dann bei 1500 rpm für 3 min zentrifugiert. Nach dem Waschen mit PBS werden die Zellen für 15 min wie im Beispiel 1 mit einer Trypsin-Lösung inkubiert.

Anschließend werden die Pellets in folgenden Medienzusammensetzungen inkubiert:
Osteogenic Differentiation Medium SH30881.02 von AdvanceSTEM mit 10% AdvanceSTEM Cell Growth Nr. SH30878.02. Die Zellen wurden bis zu einer Konfluenz von 70% in Petrischalen herangezogen und dann weiter kultiviert. 50% des Mediums wurde alle drei Tagen während der ein- bis zweiwöchigen Differenzierungsperiode ausgetauscht. Osteoblasten konnten durch Färbung mit Alizarin Rot nach sieben Tagen detektiert werden.

### Beipiel 3.4: Differenzierung zu neuralen Zellen

Spheroide rFSC Zellen werden mit PBS Puffer gewaschen und bei 1500 rpm für 3 min zentrifugiert. Nach dem Waschen mit PBSW werden die Zellen mit einer Trypsin-Lösung (10fach) bei 37°C für 15 min inkubiert. (siehe Beispiel 3.2)

Die erhaltenen Pellets werden resuspendiert in Complete Culture Medium Neural Differentiation Medium, erhältlich unter der Bestellnummer SH30889.02 von AdvanceSTEM mit 10% AdvanceSTEM Cell Growth Medium SH30878.02. Die Zellen werden bis zu 70% Konfluenz in Petrischalen herangezogen und weiter kultiviert. 50% des Mediums werden jeden dritten Tag während ein- bis zweiwöchigen Differenzierungsperiode gewechselt. Neurale Zellen, die S100beta und Neurofilamente exprimieren, konnten nach etwa sieben Tagen detektiert werden.

### Beispiel 3.5: Differenzierung zu Cardiomyocyten

Enhanced Green Fluorescence (EGFP) exprimierendes spheroide rFSC wurden mit neonatalen Raten-Cardiomyocyten co-cultiviert. Die Zellen wurden alle zwei Tagen mit frischem Medium versorgt, das folgende Zusammensetzung hatte:
- DMEM (Gibco)
- 20% Foetal bovine serum (Biochrom AG)
- Penicillin G (100 U/ml)
- Streptomycin (100 mg/mL) (Gibco).

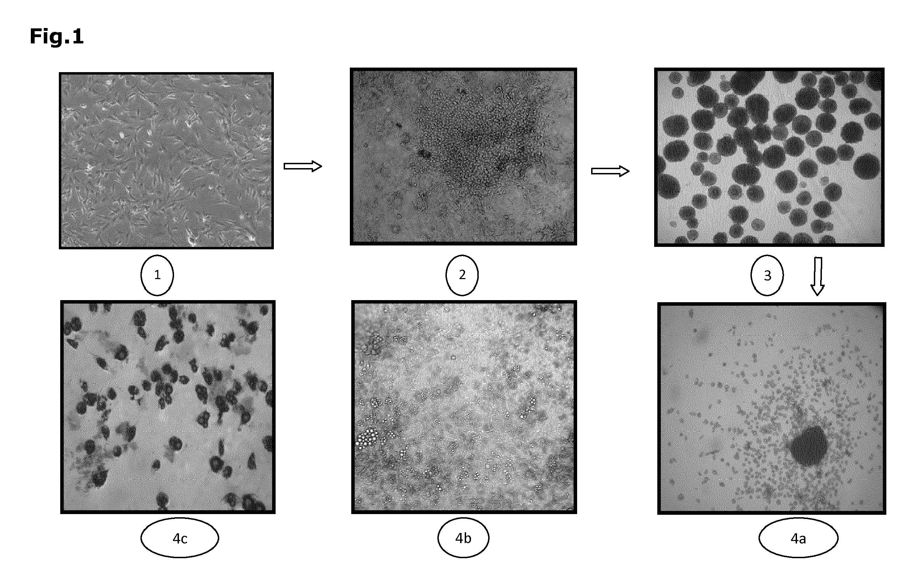

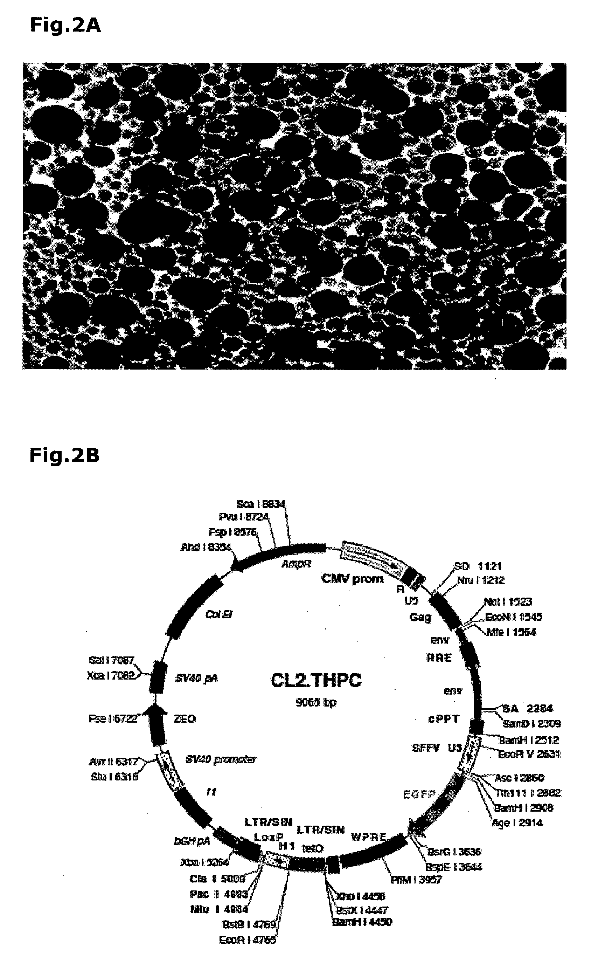

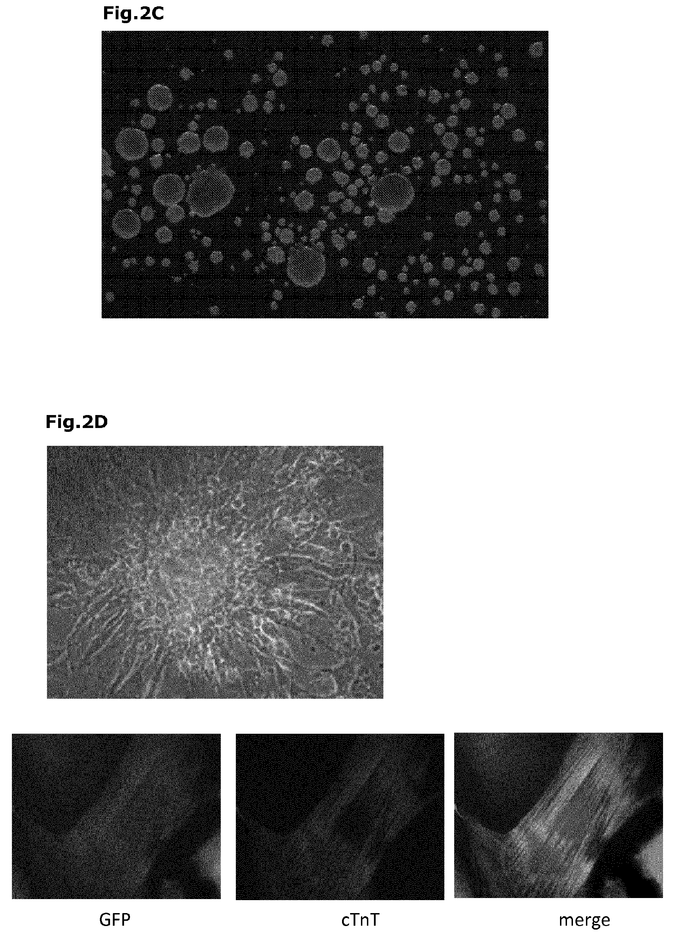

Nach drei Tagen werden einige differenzierte, grüne spheroide rFSC sichtbar. Cardiomyocyten Muskelzellen können nach sieben Tagen detektiert werden. Hierzu werden die Zellen mit 4% Paraformaldehyd fixiert und mit Hilfe von Antikörpern gegen Troponine T angefärbt. Aus Figur 2D wird ersichtlich, dass die Expression von GFP und Troponin T überlagert ist.

### Beispiel 4: Epigenetische Untersuchung

Die DNA-Methylierung der *OCT4*-5' Region von FSC 8/25 Zellen und der daraus erhaltenen spheroiden multipotenten somatischen Stammzellen wurden untersucht.

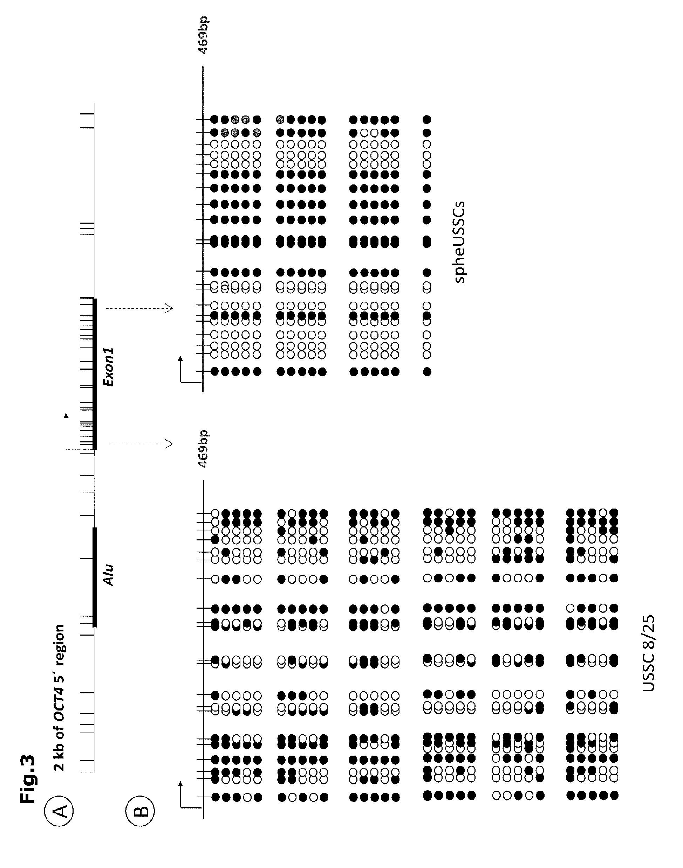

Figur 3A zeigt ein Schema der genomischen Organisation der *OCT4*-5'Region inklusive der relativen Position eines ALU-Elements, der Transkriptionsstartposition (→), des ersten Exons und die Positionen von CpG Dinucleotiden (vertikale Säulen).

Die gestrichelten Pfeile zeigen den Bereich, der mittels Bisulfit Analyse sequenziert wurde. Das sich ergebende DNA-Methylierungsmuster der beiden Zellen ist in Figur 3B dargestellt.

Kreise zeigen unmethylierte CpG Dinucleotide; gefüllte Kreise methylierte CpG Dinucleotide.

### Beispiel 5: Expression von OCT4

Die Expression von *OCT4* wurde mit spezifischen Antikörpern in einem Western Blot untersucht.

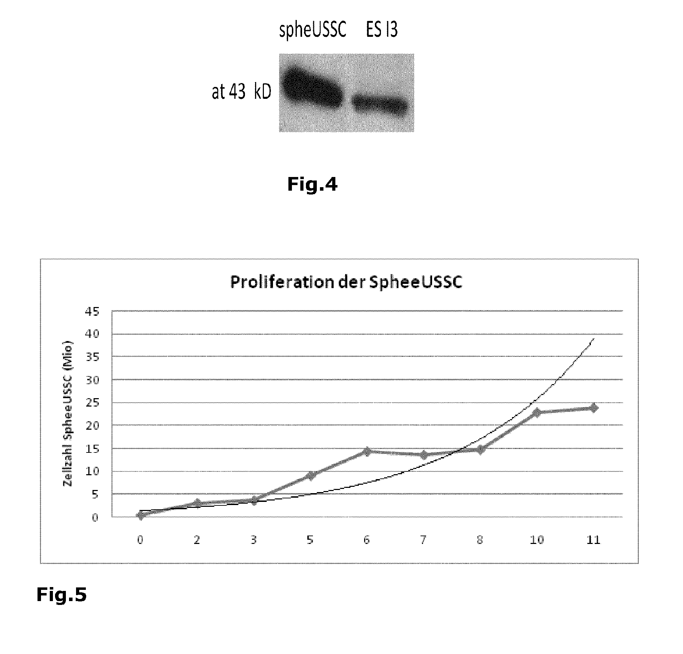

Figur 4A zeigt das Ergebnis für die erfindungsgemäßen spheroiden multipotenten somatischen Stammzellen und für die Referenz embryonalen Stammzelle I3.

### Beispiel 6: Proliferation

Die Spheroide rFSC wurden in Kultur genommen und die Proliferation beobachtet. Die Proliferationsrate beträgt: 1 Zellteilung/88 Stunden (Fig. 5). Somit liegt die Vermehrung der SpheeUSSC deutlich jenseits des Hayflick-Limits. Sie kann in Kultur für mindestens 1 Jahr stabil proliferierend gehalten werden. In dieser Zeit sind über 100 Zellteilungen nachgewiesen worden.

### Beispiel 7: Seneszenz

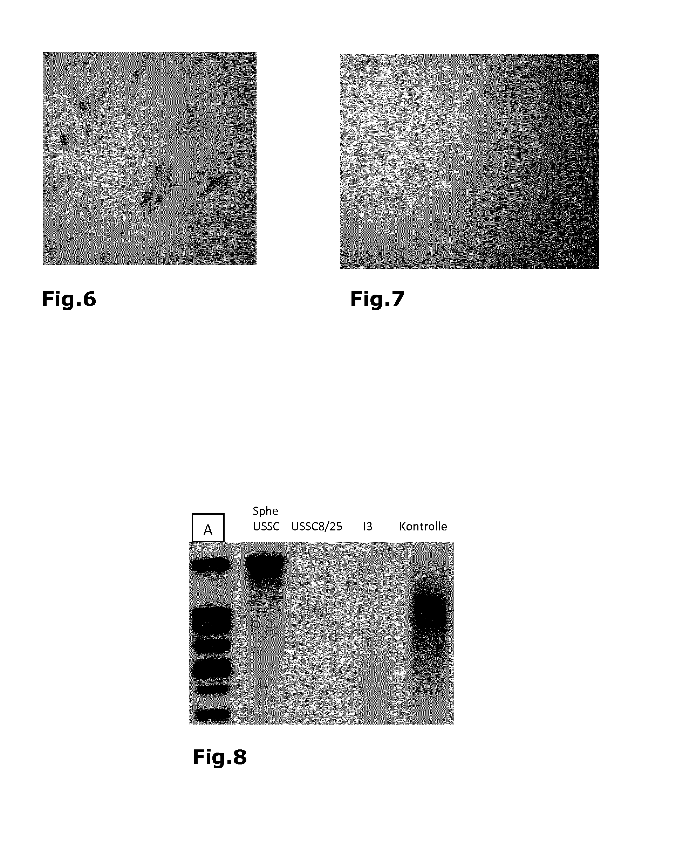

Die Bestimmung der Seneszenz mittels des Seneszenz-assoziierten β-Galaktosidase-Assays ergab im Unterschied zu vereinzelten seneszenten Zellen in der 8/25 FSC-Kultur keinen Nachweis der Seneszenz in der über ein Jahr alten Spherischen rFSC Kultur (Figuren 6 und 7).

### Beispiel 8: Telomerlänge

In Figur 8 ist die Telomerlänge TeloTAGGG Telomere Length Assay untersucht worden. Spherische rFSC zeigen lange Telomere, deutlich stärker als in der Referenz embryonalen Stammzelle I3. Keine langen Telomere konnten in FSC 8/25 und einer Kontrollprobe detektiert werden.

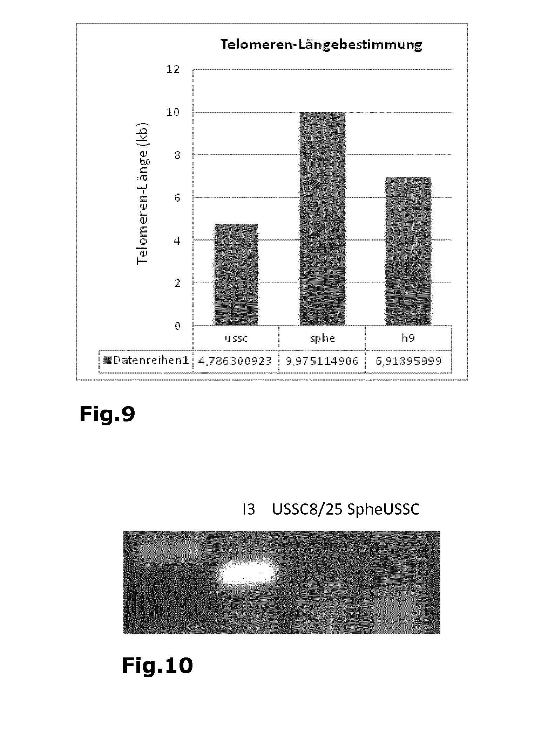

In Figur 9 ist ein PCR Nachweis nach NJ O'Callaghan and M Fenech et al. 2011 *(*A quantitative PCR method for measuring absolute telomere length. O'Callaghan NJ, Fenech M. Biol Proced Online. 2011 Jan 31;13:3*)* in FSC, rFSC und jeweils einer Referenz- embryonalen Stammzelllinie (I3, H9).

Wiederholte Versuche ein Telomerase Transkript mittels PCR in den rFSC nachzuweisen zeigten die Abwesenheit des Telomerase Transkripts (Figur. 10).

### Schlussfolgerung:

Die rFSC ist eine immortale Stammzelllinie. In ihr wurde durch die vorausgegangene epigenetische Behandlung, der ALT Mechanismus aktiviert. Somit ist sie von besonderer Bedeutung als Stammzellmodell für beispielsweise ein "drug screening".

*"ALT-positive immortalized cell lines are able to maintain their telomere length throughout many cell doublings in the absence of telomerase activity. It is generally agreed that telomere elongation in ALT cells requires a DNA recombination step. But human mesenchymal stem cells might have a particular tendency to activate ALT. "*

(Siehe: A gene expression signature classifying telomerase and ALT immortalization reveals an hTERT regulatory network and suggests a mesenchymal stem cell origin for ALT. Lafferty-Whyte K, Cairney CJ, Will MB, Serakinci N, Daidone MG, Zaffaroni N, Bilsland A, Keith WN. Oncogene. 2009 Oct 29;28(43):3765-74. doi: 10.1038/onc.2009.238.

## Patentansprüche

1. Verfahren zur Erzeugung einer spheroiden multipotenten somatischen Stammzelle umfassend die Schritte:
- Bereitstellen einer fötalen somatischen Stammzelle
- Behandlung der fötalen somatischen Stammzelle mit mindestens einem epigenetisch wirksamen Reagenz, wodurch eine spheroide multipotente somatische Stammzelle erzeugt wird.

2. Verfahren nach Anspruch 1, **dadurch gekennzeichnet, dass** das epigenetisch wirksame Reagenz eine DNA-demethylierende Eigenschaft hat.

3. Verfahren nach Anspruch 1 oder 2, **dadurch gekennzeichnet, dass** das epigenetisch wirksame Reagenz eine Histon-acetylierende Eigenschaft hat.

4. Verfahren nach einem der Ansprüche 1 bis 3, **dadurch gekennzeichnet, dass** das epigenetisch wirksame Reagenz ausgewählt wird aus 5'-Aza-2-deoxycytidin, Trichostatin-A, 5-Azacytidin, Valproinsäure, BIX-01294, Dznep und Mischungen davon.

5. Spheroide multipotente somatische Stammzelle erhältlich durch ein Verfahren nach einem der Ansprüche 1 bis 4.

6. Verfahren zur Erzeugung von Adipocyten umfassend die Schritte:
- Bereitstellen einer spheroiden multipotenten somatischen Stammzelle nach Anspruch 5;
- Kultivierung der Zelle unter geeigneten Bedingungen um Adipocyten zu erzeugen.

7. Verfahren zur Erzeugung von Cardiomyocyten umfassend die Schritte:
- Bereitstellen einer spheroiden multipotenten somatischen Stammzelle nach Anspruch 5;
- Kultivierung der Zelle unter geeigneten Bedingungen um Cardiomyocyten zu erzeugen.

8. Verfahren zur Erzeugung von Chondrocyten umfassend die Schritte:
- Bereitstellen einer spheroiden multipotenten somatischen Stammzelle nach Anspruch 5;
- Kultivierung der Zelle unter geeigneten Bedingungen um Chondrocyten zu erzeugen.

9. Verfahren zur Erzeugung von Osteoblasten umfassend die Schritte:
- Bereitstellen einer spheroiden multipotenten somatischen Stammzelle nach Anspruch 2;
- Kultivierung der Zelle unter geeigneten Bedingungen um Osteoblasten zu erzeugen.

10. Verfahren zur Erzeugung von neuronalen Zellen umfassend die Schritte:
- Bereitstellen einer spheroiden multipotenten somatischen Stammzelle nach Anspruch 2;
- Kultivierung der Zelle unter geeigneten Bedingungen um neuronale Zellen zu erzeugen.

11. Verwendung einer spheroiden multipotenten somatischen Stammzelle nach Anspruch 5 zur Erzeugung von Adipocyten, Cardiomyocyten, Chondrozyten, Osteoplasten oder neuronalen Zellen.

12. Verwendung einer spheroiden multipotenten somatischen Stammzelle nach Anspruch 5 als immortale Zelle, insbesondere zum Drug Screening.
